# EUROPÄISCHE PATENTSCHRIFT

(11) **EP 2 217 296 B1**
(45) Veröffentlichungstag und Bekanntmachung des Hinweises auf die Patenterteilung: **30.05.2012**
(21) Anmeldenummer: 08843585.4
(22) Anmeldetag: 29.10.2008
(51) Int. Cl.: A61L 17/00

(54) **Chirugisches Nahtmaterial**
Surgical suture material
Matériau de suture chirurgical

(30) Priorität: 29.10.2007 DE 102007052519
(43) Veröffentlichungstag der Anmeldung: 18.08.2010
(73) Patentinhaber: Aesculap AG, 78532 Tuttlingen/Donau (DE); HeiQ Materials AG, 5330 Bad Zurzach (CH)
(72) Erfinder: CENTONZE, Carlo, Riccardo, CH-8004 Zürich (CH); HEIGHT, Murray, CH-8006 Zürich (CH); BERNDT, Ingo, 52066 Aachen (DE); ODERMATT, Erich, CH-8200 Schaffhausen (CH)
(74) Vertreter: Baumann, Jörg
(86) Internationale Anmeldenummer: PCT/EP2008/009111
(87) Internationale Veröffentlichungsnummer: WO 2009/056281

(56) Entgegenhaltungen:
- WO-A1-2005/048708
- WO-A1-2006/032497
- WO-A1-2006/084411
- WO-A2-2007/028607
- WO-A2-2007/078304
- DE-A1-102005 044 361
- DE-A1-102006 006 675

## Beschreibung

Die vorliegende Erfindung betrifft ein medizinisches Implantat, welches mit einer antimikrobiellen Zusammensetzung ausgerüstet ist, entsprechende Herstellungsverfahren sowie die Verwendung der antimikrobiellen Zusammensetzung zur Herstellung eines medizinischen Implantats.

Post-operative Primärinfektionen und insbesondere auch Sekundärinfektionen stellen eine der größten Komplikationen in der modernen Versorgungschirurgie dar. Deswegen ist man in jüngerer Zeit zunehmend dazu übergegangen, chirurgische Implantate, insbesondere Nahtmaterialien und Netze, mit geeigneten antimikrobiellen Wirkstoffen auszustatten. Antimikrobielle Implantate dieser Art werden beispielsweise in der DE 10 2004 047 568 A1 beschrieben.

Die WO 2005/048708 A1 offenbart ein antimikrobielles und nicht zytotoxisches Schichtmaterial mit einer Biozid-Schicht und einer Transportkontrollschicht, welche Siliziumdioxid als Grundmaterial besitzen kann.

Die WO 2007/078304 A2 offenbart Kompositbeschichtungen mit Siliziumdioxid als Matrixmaterial, welches Silber- und/oder Zinkpartikel aufweisen kann.

Aus der DE 10 2006 006 675 A1 gehen antimikrobielle Verbundwerkstoffe hervor, welche ausgehend von einem siliziumorganischen Polymer und Silber- und/oder Kupfer-Nanopartikeln mittels Pyrolyse erhältlich sind.

DE 10 2005 044 361 A1 offenbart ein medizintechnisches Produkt mit einer antimikrobiellen Ausstattung aus einem Komplexmaterial aus Metallnanopartikeln und oberflächenaktiven Liganden zur Verhinderung einer Agglomeration der Partikel.

Die WO 2007/028607 A2 offenbart ein medizintechnisches Produkt mit einer antimikrobiellen Ausstattung aus einem Komplexmaterial aus Metallnanopartikeln und Polyaminosäuren.

Problematisch bei den post-operativen Infektionen sind vor allem die Sekundärinfektionen, welche häufig erst nach mehreren Wochen oder sogar Monaten auftreten können. Dies stellt vor allem bei nicht resorbierbaren Implantaten, die für einen dauerhaften Verbleib im Körper bestimmt sind, häufig ein großes klinisches Problem dar. So müssen diese Implantate gegebenenfalls wieder explantiert werden, um die aufgetretenen Infektionen erfolgreich behandeln zu können. Dadurch fallen zum Einen höhere Behandlungskosten an. Zum Anderen bedeutet jede wietere chirurgische Intervention eine zusätzliche Belastung und vor allem einen längeren Krankenhausaufenthalt für die betroffenen Patienten. Daher stellt die antimikrobielle Ausstattung von Implantaten, insbesondere von nicht resorbierbaren Implantaten, eine sinnvolle sowie notwendige Weiterentwicklung auf dem Gebiet der Implantologie dar.

Problematisch ist jedoch, dass je nach antimikrobiellen Wirkstoffen häufig eine akkumulierte, d.h. eine kurzfristige und (zu) hochdosierte, Freisetzung der Wirkstoffe in das umliegende biologische Gewebe stattfindet. Dadurch können entzündliche bis hin zu nekrotischen Gewebeveränderungen verursacht werden. Zudem kann die Integration eines Implantats im Körper durch eine akkumulierte Wirkstofffreisetzung beeinträchtigt werden.

Ein weiterer Nachteil betrifft vor allem textile Implantate, beispielsweise Nahtmaterialien, Gefäßprothesen sowie Netze und Bänder zur Hernien-, Prolaps- und Inkontinenztherapie. Dort kann es bei der Einarbeitung von Metallpartikeln, die eine bestimmte Partikelgröße überschreiten, oder durch Agglomeratbildung zu potentiellen Schwachstellen im Implantat kommen, wodurch dessen mechanische Festigkeit verringert wird. Dies gilt vor allem für feine Nahtmaterialien mit verhältnismäßig dünnen Fadendurchmessern.

Die vorliegende Erfindung stellt sich daher die Aufgabe, ein antimikrobiell ausgerüstetes Implantat bereitzustellen, welches aus dem Stand der Technik bekannte Nachteile vermeidet. Das durch die Erfindung bereitgestellte Implantat soll insbesondere einen langfristigen antimikrobiellen Schutz bieten, ohne dass hierdurch mechanische Eigenschaften des Implantats beeinträchtigt werden.

Diese Aufgabe wird erfindungsgemäß gelöst durch ein medizinisches Implantat mit den Merkmalen des unabhängigen Anspruchs 1. Bevorzugte Ausführungsformen des Implantats sind Gegenstand der abhängigen Ansprüche 2 bis 11. Die vorliegende Erfindung betrifft weiterhin auch Herstellungsverfahren für das Implantat gemäß dem unabhängigen Anspruch 12. Der Wortlaut sämtlicher Ansprüche wird hiermit durch Bezugnahme zum Inhalt dieser Beschreibung gemacht.

Bei dem erfindungsgemäßen Implantat handelt es sich um ein medizinisches Implantat, ausgerüstet mit einer antimikrobiellen Zusammensetzung, die Siliziumdioxid und metallhaltige Nanopartikel aufweist dadurch gekennzeichet, dass das Implantat ein chirugisches Nahtmaterial ist. Erfindungsgemäß kann es insbesondere vorgesehen sein, dass es sich bei der Zusammensetzung um eine antimikrobielle Zusammensetzung aus Siliziumdioxid und metallhaltigen Nanopartikeln handelt.

Durch die Erfindung wird ein antimikrobiell ausgerüstetes medizinisches Implantat bereitgestellt, dessen mechanische Eigenschaften, insbesondere dessen Festigkeit oder mechanischer Widerstand, durch die antimikrobielle Ausrüstung im Wesentlichen unbeeinflusst bleiben. Das Implantat weist weiterhin mit besonderem Vorteil einen gleichmäßigen und insbesondere lang anhaltenden antimikrobiellen Schutz auf, weswegen das Implantat auch im Hinblick auf die Vermeidung von Spätinfektionen eine leistungsstarke Alternative zu aus dem Stand der Technik bekannten Implantaten darstellt. Bei der im Rahmen der Erfindung verwendeten antimikrobiellen Zusammensetzung handelt es sich mit besonderem Vorteil um eine bioverträgliche Zusammensetzung, so dass auch eine Bioverträglichkeit des erfindungsgemäßen Implantats gewährleistet ist.

Grundsätzlich kann die antimikrobielle Ausrüstung des Implantats eine Beschichtung oder Imprägnierung des Implantats mit der Zusammensetzung bedeuten. In einer möglichen Ausführungsform ist das Implantat daher mit der antimikrobiellen Zusammensetzung beschichtet oder imprägniert, insbesondere auf der Implantatoberfläche. Die Beschichtung bzw. Imprägnierung kann zudem Bindemittel enthalten, die die Haftung zum Implantat verbessern.

Bevorzugt ist die antimikrobielle Zusammensetzung in einen Werkstoff, insbesondere in ein Polymermaterial, des Implantats eingearbeitet. Die antimikrobielle Zusammensetzung kann im Implantat fein verteilt vorliegen. Die antimikrobielle Zusammensetzung liegt im Implantat vorzugsweise dispergiert, insbesondere homogen dispergiert, vor. Erfindungsgemäß kann es zudem vorgesehen sein, dass die Zusammensetzung im Inneren des Implantats und vorzugsweise auch auf dessen Oberfläche fein verteilt ist. Durch die feine, insbesondere dispergierte, Verteilung der Zusammensetzung im Implantat ist mit besonderem Vorteil eine gleichmäßige und insbesondere kontrollierte Abgabe der antimikrobiellen Zusammensetzung, insbesondere der metallhaltigen Nanopartikel, an das umliegende Gewebe nach der Implantation möglich. Die metallhaltigen Nanopartikel können in ionogener und/oder nicht ionogener Form abgegeben werden. In der Regel werden die metallhaltigen Nanopartikel in ionogener Form abgegeben. Die ionogene Form wird gewöhnlich durch Kontakt mit Wasser oder Körperflüssigkeiten erzeugt. Ionogen vorliegende Nanopartikel zeigen dabei an ihrer Oberfläche eine antimikrobielle, insbesondere antibakterielle, Wirkung. Mit besonderem Vorteil bleibt die feine, insbesondere dispergierte, Verteilung der Zusammensetzung auch bei hohen Zusammensetzungsanteilen im Implantat erhalten.

In einer weitergehenden Ausführungsform liegen die metallhaltigen Nanopartikel im Implantat dispergiert vor. Die Nanopartikel können insbesondere im Siliziumdioxid dispergiert vorliegen. Die Nanopartikel können zumindest in der antimikrobiellen Zusammensetzung dispergiert vorliegen. Das Siliziumdioxid selbst ist vorzugsweise amorph, insbesondere röntgenamorph. Bevorzugt liegt das Siliziumdioxid in der Zusammensetzung in einer amorphen Form vor.

Die antimikrobielle Zusammensetzung an sich liegt gewöhnlich in Form eines Pulvers, insbesondere in Form eines feinen, leichten und insbesondere staubigen Pulvers, vor. Die antimikrobielle Zusammensetzung kann abhängig von den metallhaltigen Nanopartikeln eine Färbung, zumindest aber einen Farbstich, aufweisen. Beispielsweise ist die Zusammensetzung im Falle von Silbernanopartikeln leicht gelblich bis bräunlich gefärbt. Eine eventuelle Färbung der antimikrobiellen Zusammensetzung ist vor allem abhängig vom Anteil der Nanopartikel in der Zusammensetzung. Erfindungsgemäß kann es weiterhin vorgesehen sein, dass das Implantat eine durch die antimikrobielle Zusammensetzung bedingte Färbung aufweist.

Die metallhaltigen Nanopartikel liegen in einer weitergehenden Ausführungsform im Implantat, insbesondere auch im Siliziumdioxid, in Form von einzelnen, das heißt diskreten, Partikeln vor. In dieser Ausführungsform zeichnet sich das Implantat mit besonderem Vorteil dadurch aus, dass es im Wesentlichen frei von Agglomeraten oder Aggregaten der Nanopartikel ist. Dadurch kann eine negative Beeinflussung der mechanischen Stabilität des Implantats vermieden werden.

Das Siliziumdioxid weist in einer bevorzugten Ausführungsform in der antimikrobiellen Zusammensetzung eine dreidimensionale Grundstruktur, insbesondere nach Art einer Matrix, auf. Die Grundstruktur ist typischerweise durch Partikel des Siliziumdioxids gebildet. Die Grundstruktur weist mit Vorteil Hohlräume auf, die über Kanäle miteinander in Verbindung stehen können. Diese Grundstruktur, d.h. ihre Oberfläche und/oder Hohlräume bzw. Kanäle davon, weist vorzugsweise die metallhaltigen Nanopartikel auf.

Das Siliziumdioxid weist typischerweise Partikel mit einem Durchmesser zwischen 1 und 50 nm, insbesondere 5 und 30 nm, vorzugsweise 10 und 20 nm, auf. Erfindungsgemäß kann es weiterhin möglich sein, dass das Siliziumdioxid, insbesondere in der antimikrobiellen Zusammensetzung, Agglomerate aufweist. Bevorzugt weist das Siliziumdioxid Agglomerate (Aggregate) von Siliziumdioxidpartikeln auf. Besonders bevorzugt weist das Siliziumdioxid gemischte Agglomerate, insbesondere auf der Basis von Siliziumdioxidpartikeln und metallhaltigen Nanopartikeln, auf. Die Agglomerate können zu übergeordneten kettenartigen, insbesondere perlkettenartigen, Strukturen organisiert sein. Die Ketten oder kettenartigen Strukturen können weiterhin zu einer übergeordneten Struktur mit Hohlräumen und Verbindungskanälen zusammengelagert sein. Die Agglomerate besitzen vorzugsweise eine Größe, die die mechanischen Eigenschaften des Implantats, insbesondere seine mechanische Stabilität oder Festigkeit, im Wesentlichen nicht beeinflussen. Die Agglomerate weisen in der Regel einen Durchmesser zwischen 50 und 2000 nm, insbesondere 100 und 1000 nm, auf. Bei den Agglomeraten handelt es sich zudem gewöhnlich um eher lockere Verbundstrukturen, die beispielsweise unter mechanischer Belastung ohne weiteres wieder auseinander getrieben werden können. Dadurch lässt sich das Siliziumdioxid und damit vorzugsweise auch die metallhaltigen Nanopartikel besonders gut in einem Werkstoff, der zur Herstellung des Implantats verwendet wird, dispergieren.

In einer geeigneten Ausführungsform weist die antimikrobielle Zusammensetzung, insbesondere gemischte Agglomerate aus Siliziumdioxidpartikeln und den metallhaltigen Nanopartikeln, eine hohe spezifische Oberfläche, insbesondere eine spezifische Oberfläche zwischen 100 und 400 m²/g, auf.

Die Nanopartikel weisen bevorzugt einen Durchmesser zwischen 5 und 20 nm auf. Allerdings sind auch Ausführungsformen denkbar, bei welchen die Nanopartikel einen Durchmesser < 5 nm und/oder > 20 nm aufweisen.

Die metallhaltigen Nanopartikel weisen vorzugsweise einen Anteil in der antimikrobiellen Zusammensetzung zwischen 2 und 40 Gew.-%, insbesondere 5 und 20 Gew.-%, bezogen auf das Gesamtgewicht der Zusammensetzung, auf. Der Anteil an Siliziumdioxid in der antimikrobiellen Zusammensetzung liegt bevorzugt zwischen 98 und 60 Gew.-%, insbesondere 95 und 80 Gew.-%, bezogen auf das Gesamtgewicht der Zusammensetzung.

In einer weitergehenden Ausführungsform weisen die metallhaltigen Nanopartikel einen Anteil im Implantat zwischen 10 und 5000 ppm, bezogen auf das Gesamtgewicht des Implantats, auf. Höhere Anteile an metallhaltigen Nanopartikeln führen in der Regel zu keiner nennenswerten Verbesserung des antimikrobiellen Schutzes des Implantats. Allerdings können höhere Nanopartikelanteile zu einer erhöhten Waschbeständigkeit und damit generell zu einem längeren antimikrobiellen Schutz führen. Außerdem können höhere Anteile an den metallhaltigen Nanopartikeln bei Implantaten bevorzugt sein, die ein niedriges Oberflächen-Volumen-Verhältnis aufweisen. Bevorzugt weisen die metallhaltigen Nanopartikel im Implantat einen Anteil zwischen 50 und 2000 ppm, besonders bevorzugt 200 und 1500 ppm, bezogen auf das Gesamtgewicht des Implantats, auf.

Der Anteil von Siliziumdioxid im Implantat entspricht gewöhnlich in etwa dem 4- bis 20-fachen des Nanopartikelanteils im Implantat. Bevorzugt weist das Siliziumdioxid im Implantat einen Anteil zwischen 40 und 100000 ppm, insbesondere 1000 und 10000 ppm, vorzugsweise 3000 und 6000 ppm, besonders bevorzugt 4000 und 5000 ppm, bezogen auf das Gesamtgewicht des Implantats, auf.

Die metallhaltigen Nanopartikel sind in der Regel antimikrobiell wirksam bzw. weisen antimikrobielle Eigenschaften auf. Bevorzugt sind die metallhaltigen Nanopartikel aus antimikrobiell wirksamen Metallen und/oder Metallsalzen, insbesondere Metalloxiden, gebildet. So können die Nanopartikel insbesondere aus Gold, Silber, Kupfer, Zink, Titan und/oder Salzen, vorzugsweise Oxiden, dieser Metalle gebildet sein. Im Hinblick auf Titandioxid als mögliches Metalloxid ist zu sagen, dass dieses Metalloxid in der Regel nur bei Anwesenheit von Sauerstoff und Licht eine antimikrobielle Wirkung entfaltet. Besonders bevorzugt sind die Nanopartikel aus Silber, Kupfer, Silberoxid und/oder Kupferoxid gebildet.

Wie bereits erwähnt, bietet das erfindungsgemäße Implantat mit besonderem Vorteil einen antimikrobiellen Langzeitschutz. Bevorzugt bietet das Implantat einen antimikrobiellen Schutz über einen Zeitraum von mehreren Monaten bis hin zu mehreren Jahren. Im Falle sogenannter Dauerimplantate besteht ein antimikrobieller Schutz vorzugsweise zumindest über einen Zeitraum von 10 bis 15 Jahren.

Bezüglich weiterer Merkmale und Einzelheiten zum Siliziumdioxid und den metallhaltigen Nanopartikeln wird auf die Druckschriften WO 2006/084411 A1, WO 2006/084390 A1 und EP 1 889 810 A1 verwiesen.

In einer weiteren Ausführungsform ist die antimikrobielle Zusammensetzung nach einem Flammensprüh-Pyrolyseverfahren herstellbar und/oder hergestellt. Hierfür wird in einem ersten Schritt eine Lösung aus einem Metallsalz und einer vorzugsweise leicht flüchtigen Siliziumverbindung in einem organischen Lösungsmittel hergestellt. Als geeignete Siliziumverbindung kommen insbesondere organische Silane, beispielsweise Tetraethoxyorthosilan und/oder Hexamethyldisiloxan, in Betracht. Geeignete Lösungsmittel stellen Alkohole, insbesondere Methanol, Ethanol, n-Propanol, n-Butanol, iso-Propanol, Ethandiol, Propandiol sowie Mischungen davon, dar. Gegebenenfalls kann eine Ultrabeschallung durchgeführt werden, um das Auflösen des Metallsalzes in dem Lösungsmittel zu unterstützen. Die Lösung wird in einem zweiten Schritt in eine Flamme mit einer Temperatur von ca. 1500 °C versprüht. Die Flamme wird gewöhnlich mit Hilfe eines Gasgemisches, beispielsweise aus Methan und Sauerstoff, gezündet. Danach erhält sich die Flamme durch Verbrennung der Lösung selbst. Die Abscheidung der antimikrobiellen Zusammensetzung, insbesondere in Form von feinen Partikeln, erfolgt vorzugsweise an wassergekühlten Filtersystemen, an welchen zudem ein Unterdruck angelegt werden kann. Bezüglich weiterer Merkmale und Einzelheiten zu dem Flammensprüh-Pyrolyseverfahren wird auf die Druckschriften WO 2006/084411 A1, WO 2006/084390 A1 und EP 1 889 810 A1 Bezug genommen.

Das Implantat ist bevorzugt aus polymeren Werkstoffen gebildet. Bei den Werkstoffen kann es sich allgemein um Homo-, Co-, Tri- oder Tetrapolymere usw. handeln. Die Werkstoffe können insbesondere als Blockcopolymere oder Blockterpolymere vorliegen. Unter dem Begriff Werkstoff im Sinne der vorliegenden Erfindung soll der natürliche Werkstoff (Rohwerkstoff ohne die antimikrobielle Zusammensetzung) verstanden werden.

Das Implantat kann grundsätzlich aus einem resorbierbaren oder nicht resorbierbaren Werksstoff gebildet sein. Bevorzugt ist das Implantat aus einem nicht resorbierbaren Werkstoff gebildet. Nicht resorbierbare Werkstoffe sind von besonderem Vorteil, wenn ein langfristiger antimikrobieller Schutz und insbesondere eine niedrig dosierte Freisetzung von antimikrobiell wirksamen Bestandteilen der Zusammensetzung, in der Regel der metallhaltigen Nanopartikel, erreicht werden soll. Als nicht resorbierbare Werkstoffe kommen die üblicherweise zur Herstellung von nicht resorbierbaren Implantaten verwendbaren Werkstoffe, vorzugsweise Polymere, in Betracht. Als nicht resorbierbare Werkstoffe kommen vor allem Polyolefine, Polyester und in manchen Fällen auch Polyamide in Frage. Bevorzugt handelt es sich bei den nicht resorbierbaren Werkstoffen um Polyethylen, Polypropylen, Polytetrafluorethylen, Polyethylenterephthalat und/oder Polyurethan. Bei dem Polytetrafluorethylen handelt es sich vorzugsweise um expandiertes Polytetrafluorethylen.

Alternativ zu oder in Kombination mit den zuvor beschriebenen Werkstoffen ist das Implantat in einer möglichen Ausführungsform aus einem resorbierbaren Werkstoff gebildet. Als geeignete resorbierbare Werkstoffe kommen vor allem Polymere, insbesondere Co- oder Terpolymere, vorzugsweise Blockcopolymere oder Blockterpolymere, auf der Basis von Hydroxycarbonsäureeinheiten in Betracht. Bevorzugt sind Polymere auf der Basis von Lactid, Glykolid, Trimethylencarbonat, ε-Caprolacton, Paradioxanon und/oder Hydroxybuttersäure.

In einer weitergehenden Ausführungsform ist das Implantat teilweise aus einem nicht resorbierbaren Werkstoff und teilweise aus einem resorbierbaren Werkstoff gebildet. Bezüglich der in Betracht kommenden Werkstoffe wird insbesondere auf die vorhergehenden Ausführungsformen Bezug genommen.

Erfindungsgemäß kann es weiterhin vorgesehen sein, dass das Implantat neben der antimikrobiellen Zusammensetzung weitere Additive aufweist. Bei den Additiven kann es sich beispielsweise um Bindemittel, Antibiotika, entzündungshemmende Wirkstoffe, geruchsbekämpfende Wirkstoffe, Desinfektionsmittel, Wachstumsfaktoren oder dergleichen handeln. Erfindungsgemäß kann es weiterhin vorgesehen sein, dass das Implantat ein Röntgenkontrastmittel, insbesondere eine bariumhaltige Verbindung, beispielsweise Bariumsulfat, aufweist.

Gemäß einer weitergehenden Ausführungsform weist das Implantat Fäden, insbesondere Polymerfäden, mit einem Titer zwischen 3 und 3500 dtex, insbesondere 20 und 1000 dtex, vorzugsweise 50 und 250 dtex, auf. Bei den Fäden handelt es sich typischerweise um mono- und/oder multifile Fäden. Die Fäden können grundsätzlich einen Durchmesser zwischen 10 µm und 1,3 mm aufweisen. Bevorzugt liegen die Durchmesser im Bereich zwischen 0,04 und 0,90 mm. Als Fadenstärken kommen die üblicherweise verwendeten Fadenstärken in Betracht, insbesondere USP-8/0, USP-7/0, USP-6/0, USP-5/0, USP-4/0, USP-3/0, USP-2/0, USP-0, USP-1, USP-2, USP-3, USP-4, USP-5 und/oder USP-6, bevorzugt USP-8/0, USP-7/0, USP-6/0, USP-5/0, USP-4/0, USP-3/0, USP-2/0, USP-0, USP-1 und/oder USP-2.

Das Implantat kann weiterhin Fäden aufweisen, die beispielsweise eine Festigkeit zwischen 30 und 90 cN/tex, insbesondere 40 und 80 cN/tex, vorzugsweise 50 und 70 cN/tex, besitzen. Unter dem Begriff Festigkeit soll hierbei die Bruchdehnung der Fäden verstanden werden.

Es kann weiterhin bevorzugt sein, dass das Implantat porös ist, insbesondere Poren mit einer Porengröße zwischen 0,05 und 2 mm, aufweist. Das Implantat kann auch eine interkonnektierende Porosität aufweisen.

Das Implantat ist ein chirurgisches Nahtmaterial. Das Nahtmaterial kann mono- oder multifil sein. Erfindungsgemäß kann es zudem vorgesehen sein, dass es sich bei dem Implantat um ein geflochtenes, chirurgisches Nahtmaterial handelt. Bezüglich weiterer Eigenschaften und Merkmale des Nahtmaterials, insbesondere im Hinblick auf Fäden, Fadenstärken und Titer, wird auf die vorherige Beschreibung verwiesen.

Die Erfindung betrifft weiterhin ein Herstellungsverfahren für das erfindungsgemäße Implantat, wobei eine antimikrobielle Zusammensetzung, insbesondere in Form eines Masterbatches oder Compounds, umfassend Siliziumdioxid und metallhaltige Nanopartikel, in einen Werkstoff des Implantats bei dessen Herstellung zugegeben wird. Mit anderen Worten wird bei dem Verfahren die erfindungsgemäß vorgesehene antimikrobielle Zusammensetzung in einen Implantatwerkstoff unter Ausbildung des erfindungsgemäßen Implantats eingearbeitet.

Bei dem im Rahmen der vorliegenden Erfindung verwendbaren Masterbatch handelt es sich bevorzugt um ein Konzentrat der antimikrobiellen Zusammensetzung in einem polymeren Werkstoff, das in Form von Granulaten oder Pellets vorliegen kann. Vorzugsweise handelt es sich bei dem polymeren Werkstoff des Masterbatches um den Werkstoff, der zur Herstellung des Implantats verwendet wird. Die Granulate bzw. Pellets weisen gewöhnlich einen höheren Gehalt an der Zusammensetzung auf als das hergestellte Implantat.

Zur Herstellung des Masterbatches wird die antimikrobielle Zusammensetzung mit einem polymeren Werkstoff gemischt. Die Mischung wird gewöhnlich in einem Extruder aufgeschmolzen und anschließend extrudiert. Die endgültige Formgebung des Masterbatches erfolgt durch Granulieren des extrudierten Materials. Das extrudierte Material kann beispielsweise durch Stranggranulieren oder Unterwassergranulieren zu Granulaten oder Pellets weiterverarbeitet werden.

Unter einem Compound soll im Rahmen der Erfindung ein polymerer Werkstoff des Implantats verstanden werden, der bereits die gewünschte Menge an antimikrobieller Zusammensetzung enthält. In der Regel wird das Compound durch geeignete Verfahren zu dem erfindungsgemäßen Implantat weiterverarbeitet.

Gegebenenfalls können bei der Herstellung eines Masterbatches oder Compounds Dispergierhilfsstoffe zum Einsatz kommen, um eine bessere Mischung, insbesondere Dispersion, der antimikrobiellen Zusammensetzung in einem polymeren Werkstoff zu ermöglichen oder zu unterstützen. Im Übrigen sind die Techniken zur Herstellung von Masterbatches und Compounds dem Fachmann hinreichend bekannt, so dass auf eine ausführliche Beschreibung an dieser Stelle verzichtet wird.

In einer bevorzugten Ausführungsform wird die antimikrobielle Zusammensetzung mit dem Werkstoff, insbesondere einem Polymer, gemischt und anschließend zu einem erfindungsgemäßen Implantat geformt, insbesondere extrudiert, gesponnen, gepresst, gewalzt, gegossen oder geblasen. Bevorzugt wird eine Mischung aus dem Implantatwerkstoff und der antimikrobiellen Zusammensetzung, welche zweckmäßigerweise in Form eines Masterbatches vorliegt, in Form eines Fadenmaterials ausgesponnen. Als geeignete Spinntechniken kommen dabei alle dem Fachmann geläufigen Methoden in Betracht, insbesondere Schmelz-, Gel-, Lösungs- und gegebenenfalls Trockenspinnen. Bevorzugt erfolgt das Ausspinnen aus der Schmelze, welche den Werkstoff des Implantats und die antimikrobielle Zusammensetzung aufweist. Das ausgesponnene Fadenmaterial kann zu einem chirurgischen Nahtmaterial weiterverarbeitet werden. Diesbezüglich wird auf die bisherige Beschreibung Bezug genommen.

Außerdem betrifft die Erfindung ein Verfahren für die Herstellung des erfindungsgemäßen Implantats, wobei eine antimikrobielle Zusammensetzung, umfassend Siliziumdioxid und metallhaltige Nanopartikel, von außen auf ein antimikrobiell unausgerüstetes Implantat, insbesondere auf dessen Oberfläche, aufgebracht wird. Bevorzugt wird das Implantat mit der Zusammensetzung beschichtet. Zur Aufbringung der Zusammensetzung kann das antimikrobiell nicht ausgerüstete Implantat beispielsweise in ein Tauchbad, welches die Zusammensetzung enthält, eingetaucht werden. Alternativ oder in Kombination dazu kann die Zusammensetzung auch auf das Implantat aufgesprüht werden.

Das hergestellte Implantat eignet sich besonders zur Verwendung in der Chirurgie und dort insbesondere zur chirurgischen Versorgung von Wunden.

Die Erfindung betrifft schließlich auch die Verwendung einer antimikrobiellen Zusammensetzung, umfassend Siliziumdioxid und metallhaltige Nanopartikel, zur Herstellung eines erfindungsgemäßen Implantats. Bezüglich weiterer Merkmale und Einzelheiten, insbesondere in Bezug auf die Zusammensetzung, das Siliziumdioxid und/oder die metallhaltigen Nanopartikel, wird auf die bisherige Beschreibung verwiesen.

Weitere Merkmale der Erfindung ergeben sich durch die nachfolgende Beschreibung von bevorzugten Ausführungsformen anhand von Figuren und Beispielen. Hierbei können die einzelnen Merkmale der Erfindung allein oder in Kombination miteinander verwirklicht sein. Die beschriebenen Ausführungsformen dienen zur Erläuterung und zum besseren Verständnis der Erfindung und sind in keiner Weise einschränkend zu verstehen.

In den Figuren ist gezeigt:
- Figur 1:: die antimikrobiellen Eigenschaften eines erfindungsgemäßen Polypropylenfadens bei verschiedenen Silberbeladungen gegenüber verschiedenen Mikroorganismen,
- Figur: 2: (Vergleichsbeispiel, nicht unter den Umfang der Erfindung fallend.) die Freisetzungskinetik von Silber aus erfindungsgemäßen Netzen,
- Figur 3:: der Einfluss verschiedener Silberbeladungen auf die Knotenreißkraft eines erfindungsgemäßen Polypropylenfadens,
- Figur 4:: der Einfluss verschiedener Silberbeladungen auf die lineare Reißkraft eines erfindungsgemäßen Polypropylenfadens,
- Figur 5:: der Einfluss verschiedener Silberbeladungen auf die Knotenbruchdehnung eines erfindungsgemäßen Polypropylenfadens,
- Figur 6:: der Einfluss verschiedener Silberbeladungen auf die lineare Bruchdehnung eines erfindungsgemäßen Polypropylenfadens.

### Beispiel 1: Herstellung eines Masterbatches aus Silber, Siliziumdioxid und Polypropylen

In einen Doppelschneckenextruder wurden 10 Kg medical-grade Polypropylen vorgelegt und bei einer Temperatur von ca. 180 °C aufgeschmolzen. Danach wurden 250 g einer 20 Gew.-% Silber enthaltenden Zusammensetzung aus amorphem Siliziumdioxid und Silbernanopartikein dem aufgeschmolzenen Polypropylen beigemischt, so dass die Zusammensetzung in der Mischung einen Anteil von ca. 2.43 Gew.-% aufwies. Anschließend wurde die geschmolzene Mischung zu einem Granulat extrudiert. Dieses Material wies einen Silberanteil von ca. 4800 ppm auf.

### Beispiel 2: Antimikrobielle Ausrüstung eines chirurgischen Nahtmaterials

Es wurden insgesamt drei antimikrobiell ausgerüstete Nahtmaterialien hergestellt. Hierzu wurden drei Extrusionen durchgeführt, wobei vor dem Extrudieren einer Polypropylenschmelze ein entsprechend nach Beispiel 1 hergestellter Masterbatch mit einem Silberanteil von ca. 4600 ppm in verschiedenen Mengen hinzugegeben wurde.

Im ersten Ansatz wurde soviel Masterbatch hinzugegeben, dass die Mischung einen Anteil an Masterbatch von ca. 4.3 Gew.-% aufwies, bezogen auf das Gesamtgewicht des eingesetzten Rohpolymers (reines Polypropylen). Das ausgesponnene Fadenmaterial wies einen Anteil an Silber von 201 ppm auf, bezogen auf das Gesamtgewicht des Fadenmaterials.

Im zweiten Ansatz wurde soviel Masterbatch hinzugegeben, dass die Mischung einen Anteil an Masterbatch von ca. 10.9 Gew.-% aufwies, bezogen auf das Gesamtgewicht des eingesetzten Rohpolymers (reines Polypropylen). Das ausgesponnene Fadenmaterial wies einen Anteil an Silber von 421 ppm auf, bezogen auf das Gesamtgewicht des Fadenmaterials.

Im dritten Ansatz wurde soviel Masterbatch hinzugegeben, dass die Mischung einen Anteil an Masterbatch von ca. 21.7 Gew.-% aufwies, besogen auf das Gesamtgewicht des eingesetzten Rohpolymers (reines Polypropylen). Das ausgesponnene Fadenmaterial wies einen Anteil an Silber von 796 ppm auf, bezogen auf das Gesamtgewicht des Fadenmaterials.

### Beispiel 3: Antimikrobielle Tests

Ein entsprechend dem Beispiel 2 hergestelltes Nahtmaterial aus Polypropylen mit einem Silberanteil von 2600 ppm, bezogen auf das Gesamtgewicht des Nahtmaterials, wurde einem antimikrobiologischen Test gemäß des japanischen Industriestandards über die antibakterielle Aktivität und Wirksamkeit auf Textilprodukten unterworfen (JIS L 1902). Hierzu wurden ca. 0,4 g einer Probe des Nahtmaterials zu dichten Filamentbündeln gewickelt. Die Bündel wurden mit 70%igen Ethanol sterilisiert und danach getrocknet. Anschließend wurde jedes Filamentbündel mit ca. 50 µl einer Bakterienkultur geimpft, welche ca. 4 ×10⁶ kolonieformende Einheiten pro cm³ von Klebsiella pneumoniae (DSM 789) enthielt, und bei ca. 37 °C für 18 Stunden inkubiert. Anschließend wurden von jedem Filamentbündel die noch lebenden Bakterien weggespült und ausgezählt. Die hierbei erzielten Ergebnisse sind tabellarisch in Tabelle 1 aufgeführt.

**Tabelle 1**

| | Zeit [h] | CFU (Kolonie bildende Einheiten) | Verringerung der bakteriellen Kolonisierung [%] |
|---|---|---|---|
| Bakterienbeimpfte Proben ohne Ag-SiO₂-Zusammensetzung | 0 | 4.1 x 10⁶ | - |
| Unbehandelte Kontrollprobe | 18 | 4.9 x 10⁵ | - |
| Bakterienbeimpfte Proben mit der AgSiO₂-Zusammensetzung | 18 | < 9.9 × 10¹ | > 99.99 |

Weitere antimikrobielle Tests mit erfindungsgemäß ausgerüsteten chirurgischen Nahtmaterialien sind tabellarisch in Tabelle 2 aufgeführt:

**Tabelle 2**

| Polymer / Ag (ppm) | | Zeit [h] | CFU (Kolonie bildende Einheiten) | Verringerung der bakteriellen Kolonisierung [%] |
|---|---|---|---|---|
| Polystyrol /500 | Bakterienbeimpfte Proben ohne Ag-SiO₂-Zusammensetzung Unbehand. Kontrollprobe Bakterienbeimpfte Proben mit der Ag-SiO₂-Zusammensetzung | 0 | 1.5 x 10⁶ | - |
| | | nach 24h | 2.6 x 10⁷ | - |
| | | nach 24h | < 9.9 x 10¹ | > 99.99 |
| Polypropylen / 1000 | Bakterienbeimpfte Proben ohne Ag-SiO₂-Zusammensetzung Unbehand. Kontrollprobe Bakterienbeimpfte Proben mit der Ag-SiO₂-Zusammensetzung | 0 | 2.3 x 10⁵ | - |
| | | | | |
| | | nach 24h | 6.7 x 10⁶ | - |
| | | nach 24h | < 9.9 x 10¹ | > 99.99 |
| Polyamid 6/500 | Bakterienbeimpfte Proben ohne Ag-SiO₂-Zusammensetzung Unbehand. Kontrollprobe Bakterienbeimpfte Proben mit der Ag-SiO₂-Zusammensetzung | 0 | 1.5 x 10⁶ | - |
| | | nach 24h | 1.1 x 10⁷ | - |
| | | nach 24h | < 9.9 x 10¹ | > 99.99 |

### Beispiel 4: Cytotoxizität-Test

Die unter Beispiel 2 hergestellten Polypropylen-Fäden wurden einem Test auf Cytotoxizität gemäß ISO 10993 unterworfen. Die cytotoxische Wirkung wurde dabei anhand der folgenden Einstufung beurteilt:

Grad 0: keine Cytotoxizität, Grad 1: unbedeutende Cytotoxizität, Grad 2: schwache Cytotoxizität, Grad 3: moderate Cytotoxizität und Grad 4: schwerwiegende Cytotoxizität

In unten stehender Tabelle 3 sind die im Rahmen des Tests gewonnenen Ergebnisse tabellarisch aufgeführt.

**Tabelle 3**

| **Silberbeladung / ppm** | **Cytotoxizitätsgrad** |
|---|---|
| 0 | 0 |
| 201 | 0 |
| 421 | 0 |
| 796 | 0 |

Die Ergebnisse bestätigen die biokompatiblen Eigenschaften der im Rahmen der Erfindung verwendeten antimikrobiellen Zusammensetzung.

### Figurenbeschreibung

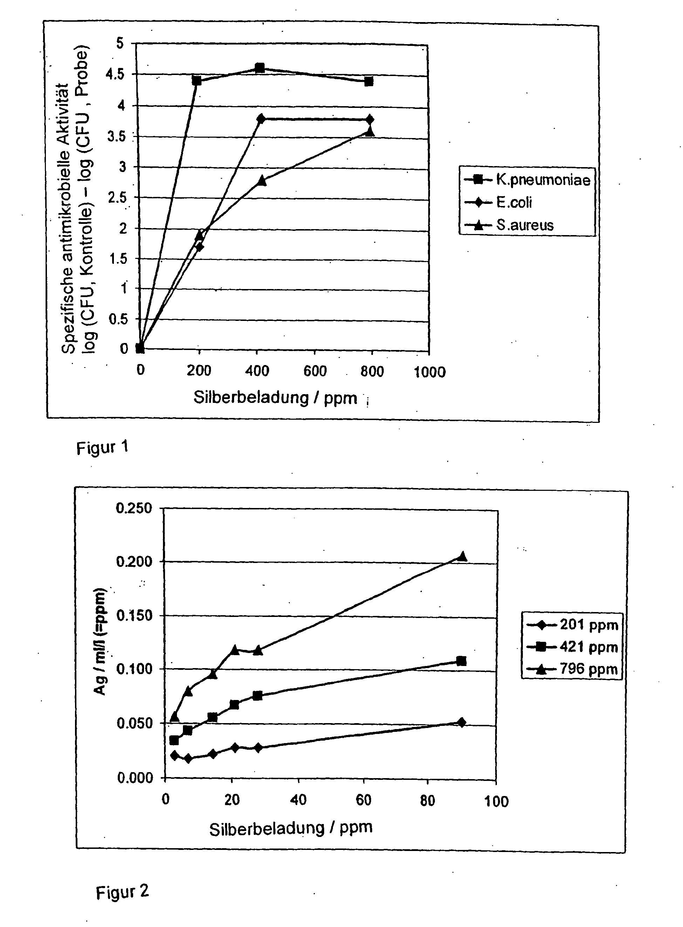

Figur 1 zeigt graphisch die antimikrobielle Wirksamkeit eines ca. 150 µm starken monofilen Polypropylenfadens, welcher gemäß der vorliegenden Erfindung antimikrobiell ausgerüstet ist, gegenüber verschiedenen Mikroorganismen. Dabei ist auf der Ordinate die antimikrobielle Aktivität als Differenz des dekadischen Logarithmus der Kolonie bildenden Einheiten (CFU: colony forming units) eines Referenzfadens und des dekadischen Logarithmus der Kolonie bildenden Einheiten (CFU: colony forming units) des antimikrobiell ausgerüsteten Fadens und auf der Abszisse die Silberbeladung (ppm) angegeben. Figur 1 zeigt eine deutliche antimikrobielle Wirksamkeit bereits bei Silberbeladungen des Fadens von deutlich < 1000 ppm. Dies gilt vor allem in Hinblick auf die Mikroorganismen Klebsiella pneumoniae und Escherichia coli, wo bereits Silberbeladungen von ca. 400 ppm zu einer hohen antimikrobiellen Aktivität des Fadens führen. Im Falle von Staphylococcus aureus und Escherichia coli konnte bei einer Silberbeladung des Fadens von ca. 800 pmm eine Abtötung > 99.9 % festgestellt werden. Im Falle von Klebsiella pneumoniae wurde, ebenfalls bei einer Silberbeladung des Fadens von ca. 800 ppm, eine Abtötung > 99.99 % beobachtet. Die Untersuchung beruhte auf der Testmethode JIS L 1902 bzw. DIN EN ISO 20743.

Figur 2 (Vergleichsbeispiel, nicht unter den Umfang der Erfindung fallend) zeigt graphisch die gemessene Freisetzungskinetik von Silber aus textilen Netzen, welche aus Fäden, wie unter Beispiel 2 beschrieben, hergestellt wurden. Zur Bestimmung der Freisetzungskinetik wurden Netzstücke (ca. 1.3 g) in 40 ml einer 0.9 % -igen NatriumchloridLösung bei 37°C extrahiert und anschließend der Silbergehalt der Lösung bestimmt. Die in Figur 2 graphisch wiedergegebenen Werte belegen eine kontinuierliche Abgabe von Silber über wenigstens 90 Tage.

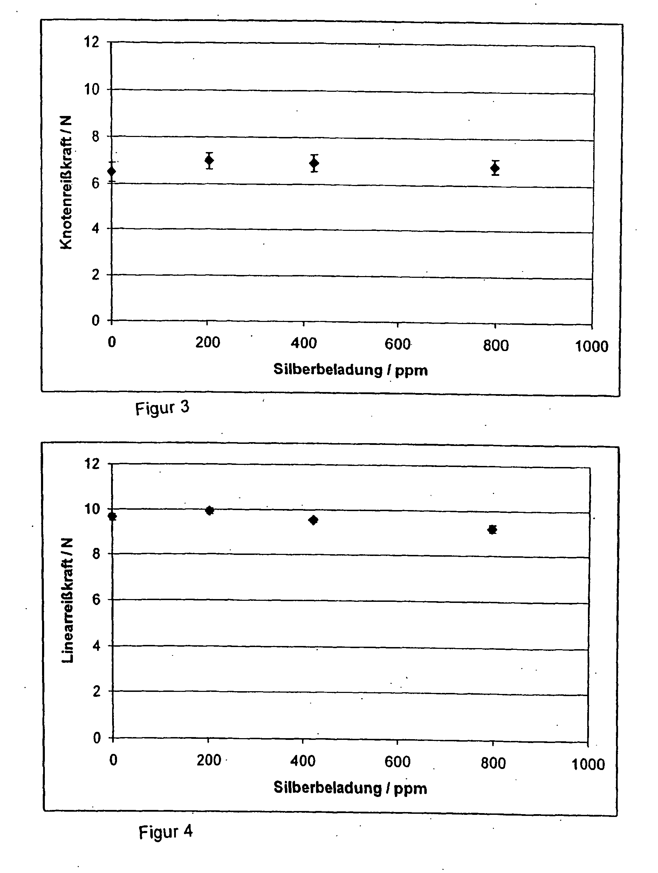

Figur 3 zeigt graphisch den Einfluss der in Beispiel 1 näher beschriebenen antimikrobiellen Zusammensetzung auf die Knotenreißkraft eines monofilen Polypropylen-Fadens mit einem Durchmesser von ca.150 µm. Dazu wurde die Knotenreißkraft (gemessen in Newton, aufgetragen auf der Ordinate von Fig. 3) in Abhängigkeit der Silberbeladung (gemessen in ppm, aufgetragen auf der Abszisse von Fig. 3) gemessen. Die Figur 3 zeigt diesbezüglich, dass die antimikrobielle Zusammensetzung zu keiner nennenswerten Veränderung der Knotenreißkraft des Polypropylenfadens führt.

Figur 4 zeigt graphisch den Einfluss der in Beispiel 1 näher beschriebenen antimikrobiellen Zusammensetzung auf die lineare Reißkraft eines monofilen Polypropylen-Fadens mit einem Durchmesser von ca. 150 µm. Dazu wurde die lineare Reißkraft (gemessen in Newton, aufgetragen auf der Ordinate von Fig. 4) in Abhängigkeit der Silberbeladung (gemessen in ppm, aufgetragen auf der Abszisse von Fig. 4) gemessen. Die in Figur 4 graphisch wiedergegebenen Daten zeigen deutlich, dass die erfindungsgemäß vorgesehene antimikrobielle Zusammensetzung auch zu keiner nennenswerten Beeinträchtigung der linearen Reißkraft des mit der Zusammensetzung ausgerüsteten Polypropylen-Fadens führt.

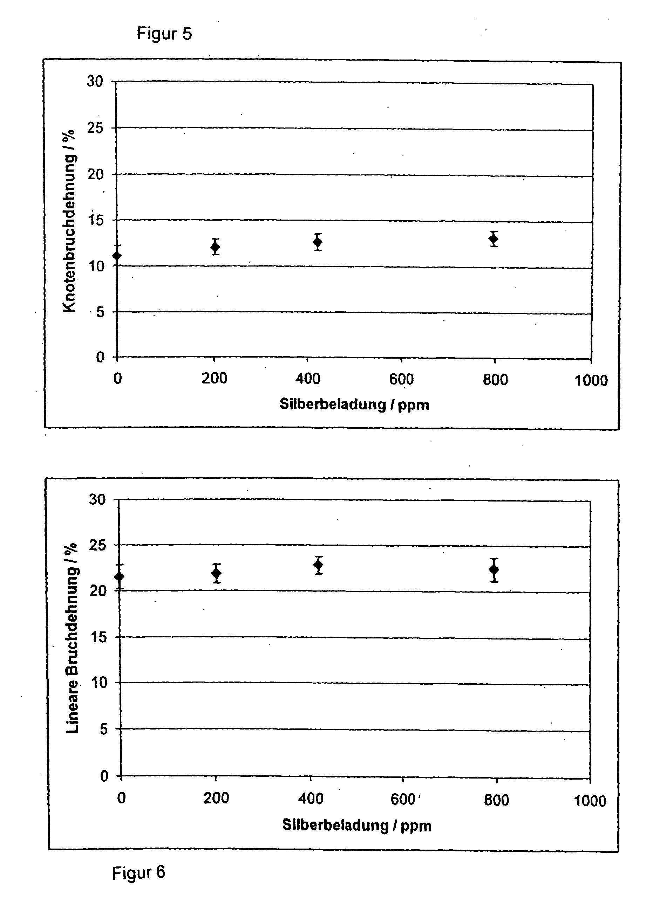

Figur 5 zeigt graphisch den Einfluss der in Beispiel 1 näher beschriebenen antimikrobiellen Zusammensetzung auf die Knotenbruchdehnung eines monofilen Polypropylen-Fadens mit einem Durchmesser von ca. 150 µm. Hierzu wurde die Knotenbruchdehnung (gemessen in Prozent, aufgetragen auf der Ordinate von Figur 5) in Abhängigkeit der Silberbeladung (gemessen in ppm, aufgetragen auf der Abszisse von Fig. 5) gemessen. Die in Figur 5 graphisch dargestellten Daten zeigen, dass die Silberbeladung des Fadens auch insoweit zu keiner nennenswerten Beeinträchtigung seiner mechanischen Eigenschaften im Vergleich zu einem silberfreien Faden führt.

Figur 6 zeigt graphisch den Einfluss der in Beispiel 1 näher beschriebenen antimikrobiellen Zusammensetzung auf die lineare Bruchdehnung eines monofilen Polypropylenfadens mit einem Durchmesser von ca. 150 µm. Hierzu wurde die lineare Bruchdehnung (gemessen in Prozent, aufgetragen auf der Ordinate von Figur 6) in Abhängigkeit der Silberbeladung (gemessen in ppm, aufgetragen auf der Abszisse von Figur 6) gemessen. Die Bruchdehnung stellt dabei ein Maß dafür dar, um welchen relativen Anteil der Faden beim Bruch gedehnt wurde. Die in Figur 6 graphisch wiedergegebenen Daten zeigen, dass die erfindungsgemäß vorgesehene antimikrobielle Zusammensetzung auch keine nennenswerte Beeinträchtigung der linearen Bruchdehnung verursacht.

Zusammenfassend lässt sich daher feststellen, dass die im Rahmen der vorliegenden Erfindung verwendbare antimikrobielle Zusammensetzung zu keiner nennenswerten Beeinträchtigung der mechanischen Eigenschaften des Fadens führt.

## Patentansprüche

1. Medizinisches Implantat, ausgerüstet mit einer antimikrobiellen Zusammensetzung, die Siliziumdioxid und metallhaltige Nanopartikel aufweist, **dadurch gekennzeichnet, dass** das Implantat ein chirurgisches Nahtmaterial ist.

2. Medizinisches Implantat nach Anspruch 1, **dadurch gekennzeichnet, dass** die antimikrobielle Zusammensetzung im Implantat dispergiert vorliegt, vorzugsweise die metallhaltigen Nanopartikel im Siliziumdioxid dispergiert vorliegen.

3. Medizinisches Implantat nach Anspruch 1 oder 2, **dadurch gekennzeichnet, dass** das Siliziumdioxid in der antimikrobiellen Zusammensetzung eine dreidimensionale Grundstruktur, insbesondere nach Art einer Matrix, aufweist.

4. Medizinisches Implantat nach einem der vorhergehenden Ansprüche, **dadurch gekennzeichnet, dass** das Siliziumdioxid in Form von Partikeln mit einem Durchmesser zwischen 1 und 50 nm, insbesondere 5 und 30 nm, vorzugsweise 10 und 20 nm, vorliegt, und vorzugsweise die metallhaltigen Nanopartikel einen Durchmesser zwischen 5 nm und 20 nm aufweisen.

5. Medizinisches Implantat nach einem der vorhergehenden Ansprüche, **dadurch gekennzeichnet, dass** Partikel des Siliziumdioxids, insbesondere zusammen mit den metallhaltigen Nanopartikeln, in Form von Agglomeraten vorliegen.

6. Medizinisches Implantat nach einem der vorhergehenden Ansprüche, **dadurch gekennzeichnet, dass** die antimikrobielle Zusammensetzung eine spezifische Oberfläche zwischen 100 und 400 m²/g aufweist.

7. Medizinisches Implantat nach einem der vorhergehenden Ansprüche, **dadurch gekennzeichnet, dass** die metallhaltigen Nanopartikel in der antimikrobiellen Zusammensetzung einen Anteil zwischen 2 und 40 Gew.-%, insbesondere 5 und 20 Gew.-%, bezogen auf das Gesamtgewicht der Zusammensetzung, aufweisen, und vorzugsweise das Siliziumdioxid in der antimikrobiellen Zusammensetzung einen Anteil zwischen 98 und 60 Gew.-%, insbesondere 95 und 80 Gew.-%, bezogen auf das Gesamtgewicht der Zusammensetzung, aufweist.

8. Medizinisches Implantat nach einem der vorhergehenden Ansprüche, **dadurch gekennzeichnet, dass** die metallhaltigen Nanopartikel im Implantat einen Anteil zwischen 10 und 5000 ppm, insbesondere 50 und 2000 ppm, vorzugsweise 200 und 1500 ppm, bezogen auf das Gesamtgewicht des Implantats, aufweisen, und vorzugsweise das Siliziumdioxid im Implantat einen Anteil zwischen 40 ppm und 100000 ppm, bezogen auf das Gesamtgewicht des Implantats, aufweist.

9. Medizinisches Implantat nach einem der vorhergehenden Ansprüche, **dadurch gekennzeichnet, dass** die metallhaltigen Nanopartikel aus Gold, Silber, Kupfer, Zink, Titan und/oder deren Salze, vorzugsweise Oxide, gebildet sind.

10. Medizinisches Implantat nach einem der vorhergehenden Ansprüche, **dadurch gekennzeichnet, dass** die Zusammensetzung nach einem Flammensprüh-Pyrolyseverfahren herstellbar oder hergestellt ist.

11. Medizinisches Implantat nach einem der vorhergehenden Ansprüche, **dadurch gekennzeichnet, dass** das Implantat Fäden mit einem Titer zwischen 3 und 3500 dtex, insbesondere 50 und 250 dtex, aufweist, und vorzugsweise das Implantat Fäden mit einer Festigkeit zwischen 30 und 90 cN/tex, insbesondere 40 und 80 cN/tex, vorzugsweise 50 und 70 cN/tex, aufweist.

12. Verfahren zur Herstellung eines medizinischen Implantats nach einem der vorhergehenden Ansprüche, **dadurch gekennzeichnet, dass** eine antimikrobielle Zusammensetzung, insbesondere in Form eines Masterbatches oder Compounds, umfassend Siliziumdioxid und metallhaltige Nanopartikel, zu einem Werkstoff des Implantats bei dessen Herstellung zugegeben oder dass eine antimikrobielle Zusammensetzung, umfassend Siliziumdioxid und metallhaltige Nanopartikel, von außen auf ein antimikrobiell unausgerüstetes Implantat aufgebracht wird.

## Claims

1. Medical implant equipped with an antimicrobial composition which comprises silicon dioxide and metal-containing nanoparticles, **characterized in that** the implant is a surgical suture material.

2. Medical implant according to Claim 1, **characterized in that** the antimicrobial composition is present dispersed in the implant, preferably the metal-containing nano-particles are present dispersed in the silicon dioxide.

3. Medical implant according to Claim 1 or 2, **characterized in that** the silicon dioxide in the antimicrobial composition has a three-dimensional basic structure, in particular of the matrix type.

4. Medical implant according to one of the previous claims, **characterized in that** the silicon dioxide is present in the form of particles with a diameter between 1 and 50 nm, in particular 5 and 30 nm, preferably 10 and 20 nm, and preferably the metal-containing nanoparticles have a diameter between 5 nm and 20 nm.

5. Medical implant according to one of the previous claims, **characterized in that** particles of the silicon dioxide, in particular together with the metal-containing nanoparticles, are present in the form of agglomerates.

6. Medical implant according to one of the previous claims, **characterized in that** the anti-microbial composition has a specific surface area between 100 and 400 m²/g.

7. Medical implant according to one of the previous claims, **characterized in that** the content of the metal-containing nanoparticles in the antimicrobial composition is between 2 and 40 wt.%, in particular 5 and 20 wt.%, based on the total weight of the composition, and preferably the content of the silicon dioxide in the antimicrobial composition is between 98 and 60 wt.%, in particular 95 and 80 wt.%, based on the total weight of the composition.

8. Medical implant according to one of the previous claims, **characterized in that** the content of the metal-containing nanoparticles in the implant is between 10 and 5000 ppm, in particular 50 and 2000 ppm, preferably 200 and 1500 ppm, based on the total weight of the implant, and preferably the content of the silicon dioxide in the implant is between 40 ppm and 100 000 ppm, based on the total weight of the implant.

9. Medical implant according to one of the previous claims, **characterized in that** the metal-containing nanoparticles are formed of gold, silver, copper, zinc, titanium and/or salts thereof, preferably oxides.

10. Medical implant according to one of the previous claims, **characterized in that** the composition is preparable or prepared by a flame spray pyrolysis process.

11. Medical implant according to one of the previous claims, **characterized in that** the implant contains fibers with a titer between 3 and 3500 dtex, in particular 50 and 250 dtex, and preferably the implant contains fibers with a strength between 30 and 90 cN/tex, in particular 40 and 80 cN/tex, preferably 50 and 70 cN/tex.

12. Process for producing a medical implant according to one of the previous claims, **characterized in that** an antimicrobial composition, in particular in the form of a master batch or compound, comprising silicon dioxide and metal-containing nano-particles, is added to a material of the implant during the production thereof or **in that** an antimicrobial composition comprising silicon dioxide and metal-containing nanoparticles is applied from outside onto a non-antimicrobially equipped implant.

## Revendications

1. Implant médical, muni d'une composition antimicrobienne, qui présente du dioxyde de silicium et des nanoparticules métalliques, **caractérisé en ce que** l'implant est un matériau de suture chirurgical.

2. Implant médical selon la revendication 1, **caractérisé en ce que** la composition antimicrobienne se trouve sous forme dispersée dans l'implant, les nanoparticules métalliques se trouvant de préférence sous forme dispersée dans le dioxyde de silicium.

3. Implant médical selon la revendication 1 ou 2, **caractérisé en ce que** le dioxyde de silicium dans la composition antimicrobienne présente une structure de base tridimensionnelle, en particulier de type matrice.

4. Implant médical selon l'une quelconque des revendications précédentes, **caractérisé en ce que** le dioxyde de silicium se trouve sous forme de particules présentant un diamètre entre 1 et 50 nm, en particulier entre 5 et 30 nm, de préférence entre 10 et 20 nm, et les nanoparticules métalliques présentent de préférence un diamètre entre 5 nm et 20 nm.

5. Implant médical selon l'une quelconque des revendications précédentes, **caractérisé en ce que** les particules du dioxyde de silicium, en particulier ensemble avec les nanoparticules métalliques se trouvent sous forme d'agglomérats.

6. Implant médical selon l'une quelconque des revendications précédentes, **caractérisé en ce que** la composition antimicrobienne présente une surface spécifique entre 100 et 400 m²/g.

7. Implant médical selon l'une quelconque des revendications précédentes, **caractérisé en ce que** les nanoparticules métalliques dans la composition antimicrobienne représentent une proportion entre 2 et 40% en poids, en particulier entre 5 et 20% en poids, par rapport au poids total de la composition et le dioxyde de silicium dans la composition antimicrobienne représentant de préférence une proportion entre 98 et 60% en poids, en particulier entre 95 et 80% en poids, par rapport au poids total de la composition.

8. Implant médical selon l'une quelconque des revendications précédentes, **caractérisé en ce que** les nanoparticules métalliques dans l'implant représentent une proportion entre 10 et 5000 ppm, en particulier entre 50 et 2000 ppm, de préférence entre 200 et 1500 ppm, par rapport au poids total de l'implant, et le dioxyde de silicium dans l'implant représentant de préférence une proportion entre 40 ppm et 100 000 ppm, par rapport au poids total de l'implant.

9. Implant médical selon l'une quelconque des revendications précédentes, **caractérisé en ce que** les nanoparticules métalliques sont formées à partir d'or, d'argent, de cuivre, de zinc, de titane et/ou de leurs sels, de préférence les oxydes.

10. Implant médical selon l'une quelconque des revendications précédentes, **caractérisé en ce que** la composition peut être préparée ou est préparée selon un procédé de pyrolyse par pulvérisation dans la flamme.

11. Implant médical selon l'une quelconque des revendications précédentes, **caractérisé en ce que** l'implant présente des fils d'un titre entre 3 et 3500 dtex, en particulier entre 50 et 250 dtex, et l'implant présentant de préférence des fils d'une résistance entre 30 et 90 cN/tex, en particulier entre 40 et 80 cN/tex, de préférence entre 50 et 70 cN/tex.

12. Procédé pour la réalisation d'un implant médical selon l'une quelconque des revendications précédentes, **caractérisé en ce qu'**une composition antimicrobienne, en particulier sous forme d'un lot-maître ou d'un compound, comprenant le dioxyde de silicium et les nanoparticules métalliques est ajoutée, à un matériau de l'implant lors de sa fabrication ou **en ce qu'**une composition antimicrobienne, comprenant du dioxyde de silicium et des nanoparticules métalliques, est appliquée à partir de l'extérieur sur un implant non apprêté de manière antimicrobienne.
